(19) Europäisches Patentamt
European Patent Office
Office européen des brevets

(11) Publication number: 0 163 094 B1

(12) **EUROPEAN PATENT SPECIFICATION**

(45) Date of publication of patent specification: 02.08.89

(51) Int. Cl.$^4$: **C 07 C 57/58**, C 07 C 57/30, C 07 C 51/08, C 07 C 51/487, C 07 B 57/00

(21) Application number: 85104646.6

(22) Date of filing: 17.04.85

(54) **Preparation of optically-active alpha-substituted phenylacetic acids.**

(30) Priority: 30.05.84 US 615392

(43) Date of publication of application: 04.12.85 Bulletin 85/49

(45) Publication of the grant of the patent: 02.08.89 Bulletin 89/31

(84) Designated Contracting States: BE CH DE FR GB IT LI NL

(56) References cited: US-A-4 209 638

(73) Proprietor: E.I. DU PONT DE NEMOURS AND COMPANY
1007 Market Street
Wilmington Delaware 19898 (US)

(72) Inventor: Friend, Peter S.
1324 Castle Court
Houston Texas 77006 (US)

(74) Representative: Dreiss, Hosenthien & Fuhlendorf
Gerokstrasse 6
D-7000 Stuttgart 1 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 163 094 B1

## Description

1. Field of the Invention

The present invention relates to a process for preparing optically-active alpha-substituted phenylacetic acids from the corresponding racemic alpha-substituted phenylacetonitriles.

2. Description of the Prior Art

It is known that alpha-substituted phenylacetic acids and derivatives thereof are themselves biologically active or are intermediates to biologically active materials, for example, in U.S. patents 3,452,079, 4,062,968 and 4,199,595. It is also known that a particular biological activity of the materials usually resides primarily in one enantiomer of the acid moiety. Accordingly, it is useful to produce only the desired enantiomer. Various methods are known to prepare alpha-substituted phenylacetic acids, to resolve these acids and even to racemize these acids, which methods result in overall complex processes to obtain a desired acid enantiomer when starting from the alpha-substituted phenylacetonitrile. For example, U.S. patents 4,062,968 and 4,220,592 described hydrolysis of alpha-substituted phenylacetonitriles, U.S. patent 2,337,352 and 4,376,213 and Japanese patents 50/25,544 and 55/136,245 describe resolution of alpha-substituted phenylacetic acids with amines and U.S. patent 4,245,116 describes racemization of alpha-substituted phenylacetic acid by forming a racemate of the alkali metal or alkaline earth metal salt at above 110°C followed by hydrolysis.

The object of the present invention is to provide a new simple process for the preparation of optically-active alpha-substituted phenylacetic acids from the corresponding racemic nitriles which elminates additional steps and reagents which had heretofore been needed for the separate process to racemize the undesired optically-active acid.

Summary of the Invention

A process of preparing a desired optically-active alpha-substituted phenylacetic acid which comprises forming a mixture of racemic alpha-substituted phenylacetonitrile and undesired optically-active alpha-substituted phenylacetic acid moiety, treating the mixture with a strong base in the presence of water, resolving the resulting racemic alpha-substituted phenylacetic acid moiety and recovering the desired optically-active alpha-substituted phenylacetic acid. Preferably, the undesired optically-active phenylacetic acid moiety from the resolution is recycled for forming the mixture with the nitrile.

Thus, the present invention provides a novel and simple process in which hydrolysis of the starting material, racemic alpha-substituted phenylacetonitrile, is conducted in the presence of simultaneous racemization of the (recycled) undesired form of the corresponding optically-active alpha-substituted phenylacetic acid. The racemic alpha-substituted phenylacetic acid moiety in the hydrolysis product which is resolved is a moiety RC(O)—0— in which R is an alpha-substituted phenylmethyl group and corresponds to the desired acid of the product. Usually this racemic moiety is obtained from hydrolysis as the metal alkali salt and is converted to the free racemic acid for resolution via the methods taught below, e.g. via amine salts.

In the preferred form of the process of the invention, a desired optically-active phenylacetic acid is prepared by forming a mixture of the corresponding racemic alpha-substituted phenylacetonitrile and the corresponding undesired optically-active alpha-substituted phenylacetic acid or alkali metal salt thereof, treating the mixture with an alkali metal hydroxide in the presence of water, resolving the resulting recemic alpha-substituted phenylacetic acid alkali metal salt by treating the salt or free acid obtained therefrom with an optically-active amine, recycling the undesired optically-active alpha-substituted phenylacetic acid or alkali metal salt thereof for forming the mixture with the nitrile and recovering the desired otically-active phenylacetic acid.

The process is initiated by forming a mixture of racemic alpha-substituted phenylacetonitrile corresponding to the desired acid product and a corresponding undesired optically-active alpha-substituted phenylacetic acid moiety and treating the mixture with a base. The base used to treat the racemic nitrile include those bases which will hydrolyze the nitrile to the racemic acid or to a derivative thereof, which derivative can subsequently be utilized to resolve the racemic acid moiety or to give free racemic acid that can be subsequently resolved. The bases used are strong bases including alkali metal hydroxides or the like.

This hydroysis process step of the invention is conducted in initially charging a reactor vessel with a racemic alpha-substituted phenylacetonitrile and optically-active alpha-substituted phenylacetic acid moiety, which are treated with the base in the presence of water. While it is usually desirable to use an excess of base, the ratio of base to nitrile can be from about 2:1 to about 1:1. Preferably, an excess, e.g. 10-30% excess, of an alkali metal hydroxide is used. The temperature for the step is chosen so that hydrolysis occurs at a desired rate. While ambient temperatures and above are used, preferably this step is conducted at an elevated temperature above about 110°C at normal or elevated pressures. Preferably, this hydrolysis treatment is conducted with an alkali metal hydroxide, such as sodium hydroxide, at temperatures of from about 150° to about 190°C and usefully under a positive pressure of up to about 250 psig and usually up to about 150 to about 220 psig. Most of this positive pressure is obtained from the course of the reaction itself.

The reaction is conducted in the presence or absence of a usually non-hydroxylic solvent. The absence of added solvent gives adequate results

and the optical presence of solvents, particularly traces of hydroxylic solvent, in the stream of recycle undesired optically-active alpha-substituted phenylacetic acid moiety, need not be avoided. Indeed, the presence of the recycle stream improves the rate of hydrolysis of the racemic nitrile.

Thus, when the recycle stream of undesired optically-active alpha-substituted phenylacetic acid is added to the racemic nitrile, the conditions of the combined racemic nitrile hydrolysis/optically-active acid racemization are substantially the same as for the nitrile hydrolysis alone. The product of the process is recovered by conventional techniques known in the art or is used directly in a resolution process.

In one embodiment, the (hot) mixture from hydrolysis containing an alkali metal salt of the racemic alpha-substituted phenylacetic acid moiety is treated directly with an optically-active primary, secondary or tertiary amine mineral acid salt which is equivalent to only one enantiomer, in an aqueous, weakly alkaline buffered solution having a pH value at which the entire acid is present in ionized form but at which no amine salt is yet deprotonated. under such conditions the resolution is directly accomplished by the precipitation of the ammonium salt of the desired optically-active alpha-substituted phenylacetic acid while the other enatiomer remains in solution. The amount of amine salt is usually at least about equimolar to the amount of alpha-substituted phenylacetic acid moiety in the mixture obtained from the nitrile hydrolysis. In this embodiment, the optically-active amine salt is a mineral acid salt, including those salts of lower alkyl ester of alpha-phenylglycine, phenylethylamine, phenylethanolamine, l-phenyl-2-dimethylamino-1, 3-propanediol, abietylamine and the like. Of these, optically-active alpha-phenylglycine methyl or ethyl ester is preferred. Such a treatment is described in U.S. patent 4,337,352, the disclosures of which are incorporated herein by reference.

In another embodiment, the (hot) mixture from hydrolysis is treated to recover the racemic optically-active alpha-substituted phenylacetic acid and if necessary to convert it to a form suitable for resolution. For example, the hot mixture from the hydrolysis is treated with a relatively strong acid optically in the presence of a solvent. Preferably, a mineral acid, e.g. hydrochloric, sulfuric or the like. On these, a strong acid, such as hydrochloric acid is preferred. Usually the amount of strong acid used is at least about equimolar to the base in the mixture from the racemic nitrile hydrolysis. When a solvent is used water, an aliphatic, cycloaliphatic or aromatic hydrocarbon or other non-hydroxylic solvent such as halogenated hydrocarbons, ethers, or the like can be used or mixtures of such solvents. Preferably, the solvent in the recovery and/or strong acid treatment is one which will also function well in the subsequent resolution step. These include hydrocarbon or non-hydroxyiic solvents and the like or mixtures thereof. Cycloaliphatic solvents include cyclohexane, methylcyclohexane or the like. Aromatic hydrocarbons include those containing from 6 to 10 carbons, for example, benzene, toluene, o-, m- or p-xylene, the trimethylbenzenes, p-ethyltoluene and the like. Aliphatic hydrocarbons include the lower alkanes including those containing 5 to 10 carbon atoms, for example, pentane, hexane, heptane, octane, nonane, decane and isomeric forms thereof.

The by-product brine is removed and the organic phase containing free racemic alpha-substituted phenylacetic acid is available for recovery of the free racemic alpha-substituted phenylacetic acid by conventional techniques, prior to resolution, or preferalby the solution of free racemic acid is used directly in the subsequent resolution.

The free racemic alpha-substituted phenylacetic acid recovered from the hydrolysis is resolved by conventional methods of resolving acids, including chemical and biochemical methods for resolving acids or derivatives of the acids, including the various methods, for example, described in Eliel, E., "Stereochemistry of Carbon Compounds", McGraw-Hill Book Co., N.Y. (1962).

Of the various methods known in the art, the chemical methods of resolution through salt formation and selective fractional precipitation or crystallization with amine is preferred.

Of these methods of using amines, resolution via conversion to diastereoisomers is usually most practical. The racemic acid is resolved into its two enantiomeric forms by salt formation with optically-active (chiral) amines to give the diastereoisomer salts. Any optically-active amine can be used, including various acyclic, carboncyclic and hetrocyclic amines, such as those specifically disclosed in Newman, P., "Optical Resolution Procedures for Chemical Compounds", Vol. 1, Amines and Related Compounds, Optical Resolution Information Center, Manhatten College, Riverdale, N.Y., Library of Congress Catalog Card No. 78-61452, U.S. patents 4,337,352, and Japanese patents 50/25,544 and 55/136,245 and the like. For example, quinine, brucine, cinchonidine, cinchonine, hydroroxyhydrindamine, morphine, alpha-phenyl-beta-phenylethylamine, alpha-phenyl-beta (p-tolyl)ethylamine, phenyloxynaphthylamine, alpha-(l-naphthyl)ehtylamine, quinidine, l-fenchylamine, strychnine, basic amino acids, such as lysine or arginine, and the like. Preferably, the optically-active amine is an aryl amine containing from 8 to 20 carbon atoms, and especially, alpha-phenyl-beta-(p-tolyl)ethylamine, alpha-phenyl-beta-phenylethylamine or alpha-phenylethylamine.

In another embodiment of the resolution, non-chiral amines are used to form a supersaturated solution of the amine salt such that the salt of one enantiomer of the alpha-substituted phenylacetic acid crystallizes out. For example, in U.S. patent 4,376,213 an alpha-substituted phenylacetic acid is resolved using diethylamine.

The resolution with chiral or non-chiral amines

is conducted by treating the racemic alpha-substituted phenylacetic acid with an amine capable of forming a salt with one enantiomer of the racemic alpha-substituted phenylacetic acid, usually in an inert solvent from which the desired (diastereoisomer) amine salt of the acid preferentially is recoverable, e.g. by precipitation, crystallization, chromatography or the like. For best results, the differential solubilities of the amine salts of the two optically-active (enantiomeric) forms of the acid in the solvent should be high. For example, the organic phase containing free racemic alpha-substituted phenylacetic acid is treated with the amine, usually in the presence of a solvent. The resulting mixture is usually heated, e.g. to reflux, followed by cooling until one of the (diastereoisomer) salts preferentially crystallizes or precipitates.

The solvent when used in resolution, precipitation or crystallization, is one from which the desired (diastereoisomer) amine salt preferential crystallizes from and is usually water, a hydroxylic or an aliphatic aromatic or cycloaliphatic hydrocarbon solvent. Suitable hydroxylic solvents include lower alkonols containing from 1 or 4 carbon atoms, such as methanol, ethanol, isopropanol, butanol and the like. Of these, methanol or ethanol are preferred. Hydrocarbon solvents include those previously described above. Preferably the solvent is an alkanol or aromatic hydrocarbon. Of these, methanol or toluene or a mixture thereof is preferred.

The preciptiation of the amine salt of the optically-active alpha-substituted phenylacetic acid is conducted with or without the addition of seed crystals of the amine salt of the desired optically-active phenylacetic acid. While it is usually desirable that essentially equimolar amounts of racemic alpha-substituted phenylacetic acid and amine are used, variations of from about 1:2 to about 2:1 are used. The temperature for this resolution step is chosen so that it is not high enough to cause decomposition of the amine salt but at which precipitation will occur. This varies depending on the particular salt, but is usually within ambient temperatures. Initially, it can be useful to heat a solution of the racemic acid and the amine to aid in salt formation and form a saturated solution at from about 50° to about 150°C. The solution is then cooled to ambient temperatures for precipitation. Precipitation proceeds normally over a period of time of up to several days. Use of lower temperatures, stirring and/or seed crystals are factors which can be used to reduce the time.

The separation and recovery of the precipitated solid or crystalline product amine salt of the optically-active alpha-substituted phenylacetic acid from the mother liquor is achieved by conventionial methods known in the art, such as filtration, or centrifugation and decantation of the mother liquor. Then, the resulting solids are usually washed with a solvent to remove any clinging mother liquor and, if desired, the salt is decomposed, e.g. with a strong acid, to recover the desired optically-active alpha-substituted phenylacetic acid in the solvent phase. The solvent phase containing the desired optically-active acid moiety may be used directly as a product or the optically-active phenylacetic acid is recovered after removal of the solvent, e.g. by distillation, evaporation or the like.

The mother liquor from the resolution, e.g. precipitation, containing the undesired optically-active alpha-substituted phenylacetic acid moiety, usually as the amine salt, is treated to recover the undesired optically-active acid moiety for recycle, e.g. by converting the amine salt back to the free acid or directly to its alkali metal salt. Usually, the mother liquor, optionally combined with wash solvent from washing the amine salt of the desired optically-active acid, is treated to decompose the amine salt and obtain free undesired optionally-active alpha-substituted phenylacetic acid for recycle. For example, the mother liquor, optionally with the wash, are fractionated and the bottoms treated to decompose the amine salt, e.g. with aqueous solution of a strong acid. The resulting organic phase containing the free undesired optically-active alpha-substituted phenylacetic acid is recycled and treated with an alkali metal hydoxide to form the metal salt prior to charging to the vessel containing the racemic alpha-substituted phenylacetonitrile, e.g. by wash treatment with aqueous solution of such metal compound, or is converted to the metal salt within the hydrolysis/racemization vessel containing the nitrile by using a high excess of charged base. In another embodiment, the amine salt of the undesired optically-active acid is treated at ambient temperature with an alkali metal hydroxide, e.g. aqueous sodium hydroxide, to form the corresponding metal salt for direct recycle of the (aqueous) metal salt solution, optionally in the presence of any solvent remaining from the resolution. Metal salt formation usually takes place at about ambient temperatures or higher, e.g. from about 10°-190°C and preferably from about 15°-60°C.

The hydrolysis/racemization process of the invention is conducted as a continuous, semi-continuous or batch process. Recovery and/recycle of reagents, such as solvents, resolving agents, acidic and basic treatment streams and the like are conventional and can be varied as known in the art to meet particular location needs and facilities.

While an initial advantage of the present process is its simplicity by providing a means of simultaneously conducting racemic nitrile hydrolysis and by-product undesired optically-active acid moiety racemization, other advantages are obtained. In particular, the hydrolysis rate is improved when conducted in the presence of the recycle acid stream and is not adversely effected by the presence of hydroxylic solvents in the recycle which might be expected to have lead to reaction products between free acid formed in the hydrolysis and the hydroxylic solvents. Lower pressures are obtained in the combined hydrolysis/racemization of the present invention as compared to even conducting the hydrolysis and the racemization as separate steps and therefore higher temperatures can be used in the combined hydrolysis resulting in shorter reaction times.

Optically-active alpha-substituted phenylacetic acids which are prepared by the process of the present invention are conventional materials generally known in the art, for example, as in U.S. patents 3,452, 079, 4,062968, 4,199,595, 4,376,213 and the like. Optically-active alpha-substituted phenylacetic acids often have biological activity themselves or are intermediates to useful derivatives, such as salts and esters, which have biological activity as, e.g. pesticides and pharmaceuticals. Optically-active alpha-substituted phenylacetic acids are also useful for resolving racemic amines into their enantiomers by classical resolution of the resulting amine salts.

Illustrative of the optically-active alpha-substituted phenylacetic acids which are prepared by the process of the present invention includes those of formula I

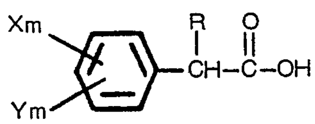
I

wherein R is an alkyl or cycloalkyl group containing up to 6 carbon atoms; each X and Y independently is a hydrogen atom; a halogen atom; an alkyl group or an alkoxy group containing from 1 to 4 carbon atoms each optionally substituted by one or more halogen atoms having an atomic number of from 9 to 35; and each m and n is an integer of 1 to 5, provided that the sum of m + n does not exceed, or X and Y when taken together form a methylenedioxy group.

In one embodiment of the invention, the optically-active alpha-substituted phenylacetic acids are those of formula I in which R is isopropyl or cyclopropyl; each X and Y independently is halogen, alkyl, haloalkyl, alkoxy or haloalkoxy in which any halogen is fluorine, bromine or chlorine and the alkyl contains 1 to 4 carbon atoms; and each m and n is 0 or 1. For example, in formula I, R is isopropyl, m is 0 and n is 1 and Y is halogen, alkyl, haloalkyl, alkoxy or haloaloxy, preferably in the para position, including optically-active alpha-isopropyl-p-chlorophenylacetic, alpha-isopropyl-p-(difluoromethoxyacetic, alpha-isopropyl-p-(tert-butyl)phenylacetic acid and the like.

Illustrative of the racemic alpha-substituted phenylacetonitrile starting materials are generally known in the art and include those of formula II

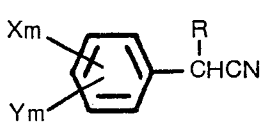
II

wherein R is alkyl or cycloalkyl group containing up to 6 carbon atoms; each X and Y independently is a hydrogen atom; a halogen atom; an alkyl group or an alkoxy containing from 1 to 4 carbon atoms each optionally substituted by one or more halogen atoms having an atomic number of from 9 to 35; and each m and n independently is an integar of 1 to 5, provided that the sum of m + n does not exceed 5, or X and Y when taken together form a methylenedioxy group. These racemic nitriles are also prepared by conventional methods know in the art to alpha-alkylate phenylacetonitriles with alkyl or cycloalkyl halides, for example, as described in U.S. patent 4,056,509.

Description of the Preferred Embodiment

A preferred embodiment of this invention is directed to a process for the preparation of optically-active alpha-isopropyl-p-chlorophenylacetic acid which comprises treating a mixture of a racemic alpha-isopropyl-p-chlorophenylacetonitrile and a corresponding optically-active alpha-substituted phenylacetic acid sodium salt with aqueous sodium hydroxide, resolving the resulting racemic alpha-isopropyl-p-chlorophenylacetic acid moiety by treating with an amine, recycling the undesired optically-active alpha-isopropyl-p-chlorophenylacetic acid moiety for racemization in the presence of the sodium hydroxide and the racemic and recovering the desired optically-active alpha-isopropyl-p-chlorophenylacetic acid moiety.

In this preferred embodiment the parameters of the steps previously discussed and the preferences thereof also apply, including any choice of solvent, temperatures, pressures, means of treatment, separation and recovery.

Illustrative Embodiments

The following embodiments are presented for the purpose of illustrating the invention and should not be regarded as limiting the invention in any way. The products were analyzed by gas chromatography using an internal standard.

Embodiment I

A reaction vessel was charged with 129 g of racemic alpha-isopropyl-p-chlorophenylacetonitrile, 225 g of 20% by weight aqueous sodium hydroxide, 40.0 g of water and 100 g of sodium R-alpha-isopropyl-p-chlorophenylacetate (89.7% R-form). The resulting mixture was heated to 180°C for 5 hours at a positive pressure of 130 psig, primarily generated by the reaction itself. Toluene was added to the hot mixture from this combined racemic nitrile hydrolysis/optically-active acid racemization and the resulting solution was neutralized with aqueous 32% hydrochloric acid. The organic phase containing free racemic alpha-isopropyl-p-chlorophenyl acetic acid was separated and the acid was recovered and to obtain R,S-alpha-isopropyl-p-chlorophenylacetic acid, 99.5% pure and essentially racemic.

Embodiment 2

Initially, in a suitable reaction vessel, 129 g of racemic alpha-isopropyl-p-chlorophenylaceto-

nitrile (CPIN) which is treated with an excess of a 20% by weight of aqueous sodium hydroxide solution at 180°C and a positive pressure of 130 psig primarily generated by the reaction itself. Toluene is added to the hot mixture from the hydrolysis to form a mixture of racemic sodium alpha-isopropyl-p-chlorophenylacetate (CPIANa). The resulting mixture is treated with 32% hydrochloric acid (HCl). The organic phase containing free racemic alpha-isopropyl-p-chlorophenylacetic acid is separated and a 1:1 methanol/toluene solution added to it. The resulting mixture containing 16% by weight of alpha-isopropyl-p-chlorophenylacetic acid is treated with 0.65 mole of (S)-phenylethylamine (PEA) in toluene per mole of alpha-isopropyl-p-chlorophenylacetic acid. The reaction mixture is heated to reflux conditions (ca 68°C) for 2 hours. After cooling at about 16°C over 5 hours, the salt of S-alpha-isopropyl-p-chlorophenylacetic acid and (S)-phenylethylamine preferentially crystallized from the mother liquor. The slurry of crystals is centrifuged to separate the solid from the mother liquor. The recovered solid is washed with toluene and a slurry is prepared of 30% by weight of the solid in toluene. This slurry is treated with HCl to a pH endpoint of 4. The resulting organic phase contains the desired product S-alpha-isopropyl-p-chlorophenylacetic acid.

The mother liquor containing R-alpha-isopropyl-p-chlorophenylacetic acid amine salt and toluene wash solution from above is continually fed to a fractionator to recover a methanol/toluene azeotrope (68% by weight of methanol), which is recycled to the crystallization step. The bottoms from the fractionator are treated with aqueous 32% HCl to neutralize the excess PEA to its hydrochloride (PEA:HCl). The PEA:HCl brine solutions are mixed with toluene to form a 50% by weight PEA solution. This mixture is treated with aqueous sodium hydroxide solution to obtain a PEA solution for recycle to the crystallization.

The organic phase remaining after PEA recover containing R-alpha-isopropyl-p-chlorophenylacetic acid in toluene is treated with a cool aqueous sodium hydroxide wash to obtain sodium R-alpha-isopropyl-p-chlorophenylacetate for recycle to the nitrile hydrolysis step for racemization simultaneously with the nitrile hydrolysis.

Following procedures similar to those described in Embodiment 2 above, optically-active alpha-isopropyl-p-(difluoromethoxy)-phenylacetic, alpha-isopropylphenylacetic, alpha-isopropyl-p-methoxyphenylacetic, alpha-isopropyl-p-methylphenylacetic, alpha-isopropyl-p-(tert-butyl)phenylacetic, and alpha-cyclopropyl-o-chlorophenylacetic acids are prepared.

Embodiment 3

A mixture of 177.9 g alpha-isopropyl-p-chlorophenylacetic acid (CPIA) (22.7% S-isomer), 110.3 g racemic alpha-isopropyl-p-chlorophenylacetonitrile (CPIN), 62 g sodium hydroxide and 423 g of water was heated to 180°C for 5.5 hours. The final pressure was 168 psig. The solution was cooled and drained, affording 796.1 g of an aqueous solution of racemic sodium alpha-isopropyl-p-chlorophenylacetate (CPIANa). This hydrolysis product (790.7 g) was combined with toluene (557.2 g) and 32% aqueous HCl was added to give a final pH of the aqueous phase of less than 1. The organic phase was separated and consisted of 881.4 g of a 33.9%w solution of racemic CPIA in toluene. The recovered racemic CPIA amounted to 298.8 g. This organic phase containing racemic CPIA was charged to a stirred 2—1 glass vessel. The charge amounted to 870.9 g or 295.2 g of racemic CPIA. Methanol (622.9 g) was added and the reaction mixture was heated to relux temperature (66°C). A solution of S-phenylethylamine (PEA) (78.8 g) and 52.6 g of toluene was added over a period of one hour. After 44.5% of the PEA solution had been added, 0.35 g of preformed PEA/S-CPIA salt was added to initiate crystallization. The mixture was heated to reflux for an additional 0.5 hour, then cooled to 20°C over a period of one hour. The crystalline product was recovered by filtration, washed with toluene and dried at 40°C under reduced pressure. There was obtained 153.8 g of PEA/S-CPIA salt. The optical purity of the contained CPIA was 91.7% S-isomer. The crystallization mother liquor (1281 g) and toluene wash (131 g) were combined. A second toluene portion of toluene (511 g) was used to wash the crystalline product, but this sample was retained. A total of 2339 g of mother liquor wash from the previous run was added and the pH was adjusted to 1.8 by the addition of 22.5 g of 32% aqueous HCl. The aqueous phase, consisting of an aqueous solution of methanol, PEA-HCl and sodium chloride, was removed. A total of 2548 g of aqueous phase was obtained. The organic phase was washed with a solution of 8.1% sodium chloride in water. A total of 2346 g of wash was recovered and was used as the initial wash for the succeeding reactions. The organic phase, 792.4 g, contained 23.41%w CPIA (185.4 g CPIA). The optical purity of the recovered CPIA was 24.5% S-isomer. A sample of the organic phase, 787.0 g, was contacted with a solution of 62 g of sodium hydroxide and 423 g water. The aqueous phase was separated and used as a portion of the feed to a succeeding hydrolysis reaction. The calculated composition was 4.1%w sodium hydroxide and 30.5%w CPIANa. The residual organic phase was essentially pure toluene and can be used as the solvent to recover racemic CPIA in the succeeding hydrolysis of racemic CPIANa. A mixture of 194.2 g R-CPIA (from above), 62 g NaOH and 423 g of water obtained from the mother liquor recovery step above was combined with 105.4 g racemic CPIN and heated to 180°C for 5.5 hours. The final pressure was 160 psig. The solution was cooled and drained, affording 789.4 g of product. This hydrolysis product (787 g) was combined with toluene (559 g) and 32%w aqueous HCl was added to give a final pH of 1. A total of 228.1 g of HCl was added. The

resulting solution was phase separated, giving 873.0 g of organic phase which contained 33.9%w racemic CPIA or a total of 295 g racemic CPIA. The organic phase containing CPIA was charged to a stirred 2-liter glass vessel. The charge amounted to a total of 861.1 g of 291.8 g racemic CPIA. Methanol (616.0 g) was added and the mixture was heated to reflux temperature (66°C). A solution of 77.9 g PEA and 52 g toluene was added over a period of one hour. After 44.4% of the PEA solution had been added, 0.35 g preformed PEA/S-CPIA salt was added to the reaction mixture to initiate crystallization of the product. When the addition of the PEA solution was complete, the reaction mixture was heated to reflux temperature for an additional 0.5 hours, then cooled to 20°C over a period of one hour. The crystalline salt was recovered by filtration, washed with toluene and dried at 40°C under reduced pressure. The yield was 153.1 g of salt. The optical purity of the contained CPIA was 91.1% S-isomer. Treatment of PEA/S-CPIA salt as a slurry in toluene with hydrochloric acid yields the desired S-alpha-isopropyl-p-chlorophenylacetic acid.

**Claims**

1. A process for preparing desired optically-active alpha-substituted phenylacetic acid which comprises forming a mixture of racemic alpha-substituted phenylacetonitrile and the undesired optically-active alpha-substituted phenylacetic acid moiety, treating the mixture with a strong base in the presence of water, resolving the resulting racemic alpha-substituted phenylacetic acid moiety and recovering the desired optically-active alpha-substituted phenylacetic acid.

2. A process according to claim 1 wherein the undesired optically-active phenylacetic acid moiety from the resolution is recycled for forming the mixture with the nitrile.

3. A process according to claim 2 wherein the base is an alkali metal hydroxide.

4. A process according to claim 3 wherein the base is a sodium hydroxide.

5. A process according to claim 1 wherein the resolution is conducted by treating the racemic acid with an amine to precipitate a salt of one enantiomer of the alpha-substituted phenylacetic acid.

6. A process according to claim 5 wherein the resolution is conducted in an inert solvent, from which the amine salt of one of the enantiomers of the acid preferably precipitates.

7. A process according to claim 6 wherein the solvent is an aliphatic, cycloaliphatic, or aromatic hydrocarbon, or hydroxylic solvent or mixtures thereof.

8. A process according to claim 7 wherein the solvent is methanol, toluene or a mixture thereof.

9. A process according to claim 6 wherein the amine is an optically-active amine.

10. A process according to claim 9 wherein the optically-active amine is an arylamine containing 8 to 20 carbon atoms.

11. A process according to claim 10 wherein the aryl amine is alpha-phenylethylamine, alpha-phenyl-beta-phenylethylamine or alpha-phenyl-beta-(p-tolyl)ethylamine.

12. A process according to claim 11 wherein the aryl amine is (S)-alpha-phenylethylamine.

13. A process according to claim 5 wherein the resolution is conducted by precipitation at ambient temperatures.

14. A process according to claim 1 wherein the salt product of the treatment of the mixture of racemic nitrile and optically-active acid moiety with base is treated with a strong acid to obtain free racemic alpha-substituted phenylacetic acid.

15. A process according to claim 14 wherein the acid is a mineral acid.

16. A process according to claim 15 wherein the acid is hydrochloric acid.

17. A process according to claim 1 wherein the base treatment of the mixture of the racemic nitrile and optically-active acid moiety is conducted at a temperature above about 110°C.

18. A process according to claim 1 wherein the undesired optically-active alpha-substituted phenylacetic acid moiety is recycled in the form of an aqueous solution of its alkali metal salt.

19. A process according to claim 18 wherein the salt is the sodium salt.

20. A process according to claim 3 wherein a desired optically-active phenylacetic acid is prepared by forming a mixture of the corresponding racemic alpha-substituted phenylacetonitrile and the corresponding undesired optically-active alpha-substituted phenylacetic acid or alkali metal salt thereof, treating the mixture with an alkali metal hydroxide in the presence of water, resolving the resulting racemic alpha-substituted phenylacetic acid alkali metal salt by treating the salt or free acid obtained therefrom with an optically active amine, recycling the undesired optically-active alpha-substituted phenylacetic acid or alkali metal salt thereof for forming the mixture with the nitrile and recovering the desired optically-active phenylacetic acid.

21. A process according to claim 20 wherein the racemic nitrile is alpha-isopropyl-p-chloroacetonitrile, the alkali metal hydroxide is sodium hydroxide, the resolution is conducted with (S)-alpha-phenylethylamine in the presence of methanol or a mixture thereof with toluene, the undesired optically-active alpha-isopropyl-p-chlorophenylacetic acid is recycled in the form of an aqueous solution of its sodium salt and the desired optically-active alpha-isopropyl-p-chlorophenylacetic acid is recovered.

**Patentansprüche**

1. Verfahren zur Herstellung erwünschter optisch aktiver alpha-substituierter Phenylessigsäure, welches das Bilden einer Mischung aus racemischem alpha-substituiertem Phenylessigsäurenitril und dem unerwünschten optisch aktiven alpha-substituierten Phenylessigsäureanteil, das Behandeln der Mischung mit einer starken Base

in Gegenwart von Wasser, das Trennen des resultiergenden racemischen alpha-substituierten Phenylessigsäureanteils und das Rüchkgewinnen der erwünschten optisch aktiven alpha-substituierten Phenylessigsäure enthält.

2. Verfahren nach Anspruch 1, dadurch gekennzeichnet, daß der unerwünschte optisch aktive Phenylessigsäureanteil aus der Trennung zur Bildung der Mischung mit dem Nitril rückgeführt wird.

3. Verfahren nach Anspruch 2, dadurch gekennzeichnet, daß die Base ein Alkalimetallhydroxid ist.

4. Verfahren nach Anspruch 3, dadurch gekennzeichnet, daß die Base ein Natriumhydroxid ist.

5. Verfahren nach Anspruch 1, dadurch gekennzeichnet, daß die Trennung durch Behandeln der racemischen Säure mit einem Amin durchgeführt wird, um ein Salz eines Enantiomers der alpha-substituierten Phenylessigsäure zu präzipitieren.

6. Verfahren nach Anspruch 5, dadurch gekennzeichnet, daß die Trennung in einem inerten Lösungsmittel durchgeführt wird, aus dem das Aminsalz eines der Enantiomere der Säure bevorzugt präzipitiert.

7. Verfahren nach Anspruch 6, dadurch gekennzeichnet, daß das Lösungsmittel ein aliphatischer, ein zykloaliphatischer, oder ein aromatischer Kohlenwasserstoff oder ein Hydroxyllösungsmittel oder eine Mischung daraus ist.

8. Verfahren nach Anspruch 7, dadurch gekennzeichnet, daß das Lösungsmittel Methanol, Toluol oder eine Mischung daraus ist.

9. Verfahren nach Anspruch 6, dadurch gekennzeichnet, daß das Amin ein optisch aktives Amin ist.

10. Verfahren nach Anspruch 9, dadurch gekennzeichnet, daß das optisch aktive Amin ein Arylamin ist, das 8 bis 20 Kohlenstoffatome enthält.

11. Verfahren nach Anspruch 10, dadurch gekennzeichnet, daß das Arylamin Alpha-Phenylethylamin, Alpha-Phenyl-Beta-Phenylethylamin oder Alpha-Phenyl-Beta-(p-tolyl)Ethylamin ist.

12. Verfahren nach Anspruch 11, dadurch gekennzeichnet, daß das Arylamin (S)-Alpha-Phenylethylamin ist.

13. Verfahren nach Anspruch 5, dadurch gekennzeichnet, daß die Trennung durch Ausfällen bei Umgebungstemperatur durchgeführt wird.

14. Verfahren nach Anspruch 1, dadurch gekennzeichnet, daß das Salz-Produkt aus der Behandlung der Mischung aus racemischem Nitril und optisch aktivem Säureanteil mit einer Base mit einer starken Säure behandelt wird, um eine freie racemische alpha-substituierte Phenylessigsäure zu erhalten.

15. Verfahren nach Anspruch 14, dadurch gekennzeichnet, daß die Säure eine anorganische Säure ist.

16. Verfahren nach Anspruch 15, dadurch gekennzeichnet, daß die Säure Salzsäure ist.

17. Verfahren nach Anspruch 1, dadurch gekennzeichnet, daß die Basenbehandlung der Mischung aus dem racemischen Nitril und dem optisch aktiven Säureanteil bei einer Temperatur oberhalb etwa 110°C durchgeführt wird.

18. Verfahren nach Anspruch 1, dadurch gekennzeichnet, daß der unerwünschte optisch aktive alpha-substituierte Phenylessigsäureanteil in Form einer wässrigen Lösung seines Alkalimetallsalzes rückgeführt wird.

19. Verfahren nach Anspruch 18, dadurch gekennzeichnet, daß das Salz Natriumsalz ist.

20. Verfahren nach Anspruch 3, dadurch gekennzeichnet, daß eine erwünschte optisch aktive Phenylessigsäure durch Bilden einer Mischung aus dem entsprechenden racemischen alpha-substituierten Phenylessigsäurenitril und der entsprechenden unerwünschten optisch aktiven alpha-substituierten Phenylessigsäure oder deren Alkalimetallsalz, durch Behandeln der Mischung mit einem Alkalimetallhydroxid in Gegenwart von Wasser, durch Trennen des resultierenden racemischen alpha-substituierten Phenylessigsäure-Alkalimetallsalzes durch Behandeln des Salzes oder der daraus erhaltenen freien Säure mit einem optischen aktiven Amin, durch Rückführen der unerwünschten optisch aktiven alpha-substituierten Phenylessigsäure oder deren Alkalimetallsalz zum Bilden der Mischung mit dem Nitril und durch Rückgewinnen der gewünschten optisch aktiven Phenylessigsäure hergestellt wird.

21. Verfahren nach Anspruch 20, dadurch gekennzeichnet, daß das racemische Nitril Alpha-Isopropyl-p-Chorazetonnitril ist, das Alkalimetallhydroxid Natriumhydroxid ist, die Trennung mit (S)-Alpha-Phenylethylamin in Gegenwart von Methanol oder einer Mischung davon mit Toluol durchgeführt wird, die unerwünschte optisch aktive Alpha-Isopropyl-p-Chlorphenylessigäure in Form einer wässrigen Lösung ihres Natriumsalzes rückgeführt wird und die erwünschte optisch aktive Alpha-Isopropyl-p-Chlorphenylessigsäure wieder gewonnen wird.

**Revendications**

1. Procédé de préparation d'acides phénylacétiques alpha-substitués optiquement actifs, comprenant la formation d'un mélange de phénylacétonitrile alpha-substitué racémique et de la fraction acide phénylacétique alpha-substitué optiquement actif indésirable, le traitement du mélange avec une base forte en présence d'eau, le dédoublement de l'acide phénylacétique alpha-substitué racémique obtenu et la récupération de l'acide phénylacétique alpha-substitué optiquement actif souhaité.

2. Procédé selon la revendication 1, dans lequel l'acide phénylacétique optiquement actif indésirable, provenant du dédoublement, est recyclé pour former le mélange avec le nitrile.

3. Procédé selon la revendication 2, dans lequel la base est un hydroxyde de métal alcalin.

4. Procédé selon la revendication 3, dans lequel la base est l'hydroxyde de sodium.

5. Procédé selon la revendication 1, dans lequel

le dédoublement est conduit par traitement de l'acide racémique avec une amine pour précipiter un sel d'un énantiomère de l'acide phénylacétique alpha-substitué.

6. Procédé selon la revendication 5, dans lequel le dédoublement est conduits dans un solvant inerte, dans lequel le sel d'amine d'un des énantiomères de l'acide précipite de préférence.

7. Procédé selon la revendication 6, dans lequel le solvant est un hydrocarbure aliphatique, cycloaliphatique ou aromatique, ou un solvant hydroxylique ou leurs mélanges.

8. Procédé selon la revendication 7, dans lequel le solvant est le méthanol, le toluène ou leurs mélanges.

9. Procédé selon la revendication 6, dans lequel l'amine est une amine optiquement active.

10. Procédé selon la revendication 9, dans lequel l'amine optiquement active est une arylamine comportant 8 à 20 atomes de carbone.

11. Procédé selon la revendication 10, dans lequel l'arylamine est l'alpha-phényléthylamine, l'alpha-phényl-béta-phényléthylamine ou l'alpha-phényl-béta-(p-tolyl)éthylamine.

12. Procédé selon la revendication 11, dans lequel l'arylamine est la (S)-alpha-phényléthylamine.

13. Procédé selon la revendication 5, dans lequel le dédoublement est conduit par précipitation à la température ambiante.

14. Procédé selon la revendication 1, dans lequel le sel produit par le traitement du mélange de nitrile racémique et de la fraction acide optiquement active avec une base traité avec un acide fort pour obtenir un acide phénylacétique alpha-substitué racémique libre.

15. Procédé selon la revendication 14, dans lequel l'acide est un acide minéral.

16. Procédé selon la revendication 15, dans lequel l'acide est l'acide chlorhydrique.

17. Procédé selon la revendication 1, dans lequel le traitement du mélange du nitrile racémique et de la fraction acide optiquement active est conduit à une température supérieure à environ 110°C.

18. Procédé selon la revendication 1, dans lequel la fraction acide phénylacétique alpha-substitué optiquement actif indésirable est recyclée sous la forme d'une solution aqueuse de son sel de métal alcalin.

19. Procédé selon la revendication 18, dans lequel le sel est le sel de sodium.

20. Procédé selon la revendication 3, dans lequel l'acide phénylacétique optiquement actif souhaité est préparé par formation d'un mélange du phénylacétonitrile alpha-substitué racémique correspondant et de la fraction acide phénylacétique alpha-substitué optiquement actif indésirable ou de son sel de métal alcalin, traitement du mélange avec un hydroxyde de métal alcalin en présence d'eau, dédoublement du sel de métal alcalin de l'acide phénylacétique alpha-substitué racémique obtenu par traitement du sel ou de l'acide libre obtenu à partir de celui-ci avec une amine optiquement active, recyclage, de l'acide phénylacétique alpha-substitué optiquement actif indésirable ou de son sel de métal alcalin pour former le mélange avec le nitrile et récupération de l'acide phénylacétique optiquement actif souhaité.

21. Procédé selon la revendication 20, dans lequel le nitrile racémique est l'alpha-isopropyl-p-chloroacétonitrile, l'hydroxye de métal alcalin est l'hydroxyde de sodium, le dédoublement est conduit avec la (S)-alpha-phényléthylamine en présence de méthanol ou d'un mélange de celui-ci avec le toluène, l'acide alpha-isopropyl-p-chlorophénylacétique optiquement actif indésirable est recyclé sous la forme d'une solution aqueuse de son sel de sodium et l'acide alpha-isopropyl-p-chlorophénylacétique optiquement actif désiré est récupéré.